(19) Europäisches Patentamt
European Patent Office
Office européen des brevets

(11) EP 4 421 065 B1

# (12) EUROPEAN PATENT SPECIFICATION

(45) Date of publication and mention of the grant of the patent:
**12.08.2026 Bulletin 2026/33**

(21) Application number: **22885769.4**

(22) Date of filing: **19.10.2022**

(51) International Patent Classification (IPC):
***C07D 213/16*** *(2006.01)* ***C07D 213/12*** *(2006.01)*
***C07C 211/55*** *(2006.01)* ***C07C 209/02*** *(2006.01)*
***B01J 29/70*** *(2006.01)*

(52) Cooperative Patent Classification (CPC):
(C-Sets available)
**B01J 29/70; C07C 209/02; C07D 213/16;**
Y02P 20/584 (Cont.)

(86) International application number:
**PCT/CN2022/126220**

(87) International publication number:
**WO 2023/071891 (04.05.2023 Gazette 2023/18)**

## (54) METHOD FOR SELECTIVELY AND CONTINUOUSLY PRODUCING 2-METHYLPYRIDINE AND DIPHENYLAMINE FROM ANILINE

VERFAHREN ZUR SELEKTIVEN UND KONTINUIERLICHEN HERSTELLUNG VON 2-METHYLPYRIDIN UND DIPHENYLAMIN AUS ANILIN

PROCÉDÉ DE PRODUCTION SÉLECTIVE ET CONTINUE DE 2-MÉTHYLPYRIDINE ET DE DIPHÉNYLAMINE À PARTIR D’ANILINE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **31.10.2021 CN 202111278790**

(43) Date of publication of application:
**28.08.2024 Bulletin 2024/35**

(73) Proprietors:
- **China Petroleum & Chemical Corporation Beijing 100728 (CN)**
- **Sinopec Dalian Research Institute of Petroleum and Petrochemicals Co., Ltd. Lushunkou District Dalian, Liaoning 116045 (CN)**

(72) Inventors:
- **LI, Haomeng Dalian, Liaoning 116045 (CN)**
- **ZHAO, Xiangyu Dalian, Liaoning 116045 (CN)**
- **WANG, Libo Dalian, Liaoning 116045 (CN)**
- **QI, Wenbo Dalian, Liaoning 116045 (CN)**
- **WANG, Zhenyu Dalian, Liaoning 116045 (CN)**
- **LI, Lanpeng Dalian, Liaoning 116045 (CN)**
- **AI, Fubin Dalian, Liaoning 116045 (CN)**

(74) Representative: **karo IP Patentanwälte PartG mbB Steinstraße 16-18 40212 Düsseldorf (DE)**

(56) References cited:
**CN-A- 1 144 796**
**CN-A- 105 384 683**
**CN-A- 106 631 829**
**JP-A- 2001 172 230**
**US-A- 4 388 461**
**US-A- 4 395 554**
**US-A- 4 395 554**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **TSCHUMPER, A. PRINS, R.: "The zeolite-catalysed isomerization of aniline to a-picoline", APPLIED CATALYSIS A: GENERAL, vol. 172, no. 2, 14 September 1998 (1998-09-14), AMSTERDAM, NL , pages 285 - 294, XP004271490, ISSN: 0926-860X, DOI: 10.1016/S0926-860X(98)00125-2**
- **SONG, LIZHI ET AL.: "A Novel Diphenylamine Synthesis Catalyst", INDUSTRIAL CATALYSIS, vol. 11, no. 3, 31 March 2003 (2003-03-31), pages 35 - 38, XP009545838, ISSN: 1008-1143**


(52) Cooperative Patent Classification (CPC): (Cont.)

C-Sets
**C07C 209/02, C07C 211/55**

## Description

### CROSS-REFERENCE TO RELATED APPLICATIONS

The application claims the benefit of Chinese patent application No. "202111278790.6", filed on October 31, 2021.

### TECHNICAL FIELD

The invention belongs to the technical field of the fine chemical industry and particularly relates to a method for synthesizing 2-methylpyridine from aniline and a method for selectively and continuously producing 2-methylpyridine and diphenylamine from aniline.

### BACKGROUND ART

2-methylpyridine, represented by the molecular formula $C_6H_7N$, also called $\alpha$-methylpyridine or $\alpha$-picoline, is one kind of pyridine base, it is a colorless oily liquid at room temperature and has a strong unpleasant odor, it has a freezing point of -38.9°C and a boiling point of 129.5°C as well as strong toxicity, and is mutually soluble with solvents such as acetone, ethanol, ether, and water. 2-methylpyridine is an important chemical intermediate and an important starting material in the fine chemical industry, can be used for producing long-acting sulfanilamide, pesticide intermediates, and feedstuff intermediates, and can also be used for synthesizing special resin vinyl pyridine and 2-vinyl pyridine intermediates. The used amount of 2-methylpyridine in China increases at a rate of about 25% per year, and thus the domestic market potential of 2-methylpyridine is tremendous.

The production methods of pyridine base in the industrial field mainly comprise a coal tar separation method and a chemical synthesis method. The coal tar separation method has the defects such as serious pollution, a few product types, high investment and costs, poor quality, high energy consumption, and the yield of the product is low and can hardly be improved. The chemical synthesis method usually adopts an ammonium aldehyde condensation method and uses aldehyde, ketone, and ammonia as starting materials to react and synthesize the pyridine base. However, the products of the ammonium aldehyde condensation method include 3-methylpyridine, 2-methylpyridine, 4-methylpyridine, and the like, the method has the problems such as difficulty in separating subsequent products.

CN105384683A discloses a method of separating byproducts 2-methylpyridine and 4-methylpyridine from the process of synthesizing diphenylamine with aniline, the steps are as follows: the aniline flows through a fixed bed reactor containing a molecular sieve catalyst, the reactor temperature and pressure are controlled; by the condensation reaction, the aniline is converted into a mixture reaction solution comprising diphenylamine, ammonia, and byproducts such as 2-methylpyridine, 4-methylpyridine, water, acridine, and 4-aminobiphenyl, the low boiling point mixed fraction is obtained by multistage distillation of the mixture reaction solution, the low boiling point mixed fraction is dehydrated, the dehydrated mixture is dried, the dried mixture is distilled to obtain 2-methylpyridine and 4-methylpyridine according to different boiling points. The essence of the method is a process of separating byproducts 2-methylpyridine and 4-methylpyridine from the traditional process of synthesizing diphenylamine with aniline. Therefore, given that the 2-methylpyridine is a byproduct in the reaction process, the yield is low, and the method is not suitable for large-scale production.

CA1190928A discloses a method for producing $\alpha$-picoline from aniline, wherein the starting material is passed over an acid zeolite catalyst in the presence of inert gas (e.g., nitrogen gas and helium gas) at the temperature range of 200-650°C to prepare a product containing alpha-picoline, the reaction pressure is from atmospheric up to 25,000kPa, usually at 200kPa, and the space velocity of aniline is preferably 0.2-5 WHSV. The acidic zeolite is preferably in the hydrogen form and includes beta-zeolite having a silica: alumina ratio of 10-100, up to 150. Wherein Example 1 uses the pure HZSM-5, the conversion rate of aniline is 13.1% and the selectivity of $\alpha$-picoline is 51.6% under the conditions consisting of the temperature of 510°C, the pressure of 2,860kPa, ammonia gas accounts for 1.5mol% of aniline, and the space velocity of nitrogen gas is 100GHSV.

"The zeolite-catalysed isomerization of aniline to $\alpha$-picoline [J]. Applied catalysis A: General 172(1998)285-294" discloses a method for preparing $\alpha$-picoline by means of an aniline rearrangement reaction, the method uses a molecular sieve, preferably Ga-MFI molecular sieve (better than ZSM-5), as the catalyst, and performed under the conditions consisting of a temperature of 673K, a total pressure of 75bar, and an ammonia partial pressure of 5-60bar, preferably 20-25bar. Wherein the selectivity of the $\alpha$-picoline can reach 85% or more, but the conversion rate is only 3.7%.

In US 4 395 554 A an Alpha-picolines are produced by containing aniline or substituted anilines, such as alkyl-, cyclo alkyl-, halo- ; alkoxy- or substituted alkyl-anilines with a crystalline zeolite catalyst having a silica:alumina ratio of at least 12 and a constraint index of 1 to 12. ZSM-5 type catalysts are contacted at elevated temperature to yield alpha-picolines selectively.

US 4 388 461 A relates to Alpha-picoline which is produced by the ammoniation of phenol over a zeolite having a

silica:alumina ratio of at least 12 and a constraint index of 1 to 12. The preferred zeolite is ZSM-5. Aniline is produced as a co-product and the alpha-picoline may be separated by distillation or extraction.

## SUMMARY OF THE INVENTION

The invention aims to overcome the defects in the prior art that the method for preparing 2-methylpyridine by using the aniline rearrangement reaction has a low conversion rate of aniline or the low selectivity of 2-methyl pyridine and provides a method for selectively producing diphenylamine and 2-methylpyridine based on the existing device for producing diphenylamine from aniline. The method of the invention can conveniently and effectively realize the switching between the diphenylamine product and the 2-methylpyridine product by controlling the temperature, the reaction conditions are mild, the yield of 2-methylpyridine is high, the reaction reactivity is desirable, thus the method has excellent industrial application prospect.

The inventors of the present invention have discovered that both the reaction for generating 2-methylpyridine by isomeric rearrangement of aniline and the reaction for preparing diphenylamine by condensation of aniline are influenced by two factors of the catalyst and the reaction temperature. The invention uses a β zeolite catalyst carrying an active metal component selected from at least one of W, Mo, Ni, and Co, the reaction route of aniline can be switched between 2-methylpyridine and diphenylamine due to the change of the catalytic activity center. In addition, the lower reaction temperature is more beneficial to the synthesis of 2-methylpyridine. Based on the finding, the present invention proposes a method for producing 2-methylpyridine and a method for selectively and continuously producing diphenylamine and 2-methylpyridine.

The first aspect of the invention provides a method for selectively and continuously producing 2-methylpyridine and diphenylamine from aniline, the method comprises: in a hydrogen-containing atmosphere, enabling an aniline and a catalyst to undergo a contact reaction at a temperature of 100-400°C, wherein the catalyst is a β zeolite carrying a metal component, and the metal component comprises an active metal component selected from at least one of W, Mo, Ni, and Co; controlling the temperature of the contact reaction to be 100-240°C to obtain a product mainly consisting of 2-methylpyridine; and controlling the temperature of the contact reaction to be 260-400°C to obtain a product mainly consisting of diphenylamine.

The second aspect of the present invention provides a method for synthesizing 2-methylpyridine from aniline, wherein the method comprises carrying out a contact reaction between aniline and a catalyst under a hydrogen-containing atmosphere and a condition for isomeric rearrangement reaction condition, the catalyst is a β zeolite carrying a metal component comprising an active metal component selected from at least one of W, Mo, Ni, and Co, and the temperature of the contact reaction is within the range of 100-240°C.

The method in the invention is used for producing 2-methylpyridine, it has the advantages of a high conversion rate of aniline and high selectivity of the target product 2-methylpyridine. The possible reasons may be deduced as follows: under a hydrogen-containing atmosphere, the reaction mechanism is greatly changed due to the selection of a β zeolite catalyst containing at least one active metal of W, Mo, Ni, and Co, thermodynamic factors become one of the factors influencing the selectivity of 2-methy pyridine, the reaction of generating one diphenylamine molecule by condensing two aniline molecules is the main reaction at a high temperature, and by reducing the temperature, the reaction is switched to the main reaction of generating 2-methy pyridine by isomeric rearrangement of the aniline molecules. Therefore, the method of the invention can realize the purpose of producing 2-methylpyridine by using aniline as a starting material and selectively producing diphenylamine and 2-methylpyridine on the existing process device of diphenylamine.

In the prior art, a large amount of ammonia gas is generally introduced for synthesizing pyridine base compounds. The presence of ammonia gas will occupy the acidic site of the catalyst, resulting in reduced catalyst activity. At the same time, the high temperature and high pressure also limit the industrial application of the process. The method provided by the invention only needs to introduce a small amount of hydrogen gas without introducing a large amount of ammonia gas, and the reaction conditions are mild, only the aniline is used as the reaction starting material, the high-purity 2-methylpyridine and high-purity diphenylamine can be obtained through the subsequent distillation operation.

## BRIEF DESCRIPTION OF THE DRAWINGS

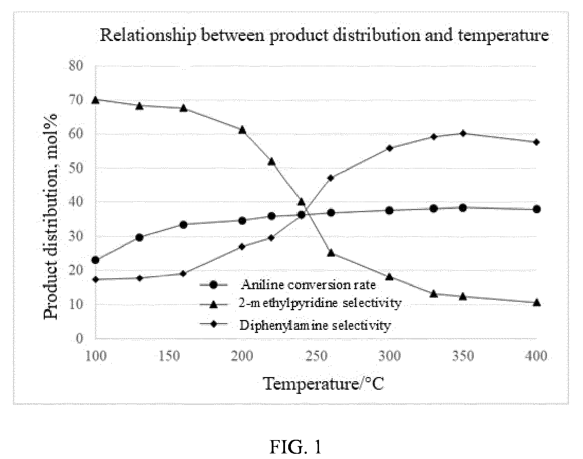

FIG. 1 is a graph showing the relationship between the product distribution of a method for selecting and continuously producing 2-methylpyridine and diphenylamine from amine provided by the present invention along with the change of temperature.

## DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical

ranges, the endpoint values of the various ranges, the endpoint values and the individual point value of the various ranges, and the individual point values may be combined with one another to produce one or more new numerical ranges, which should be deemed have been specifically disclosed herein.

The invention provides a method for synthesizing 2-methylpyridine from aniline, the method comprises carrying out a contact reaction between aniline and a catalyst under a hydrogen-containing atmosphere and a condition for isomeric rearrangement reaction, the catalyst is a β zeolite carrying a metal component comprising an active metal component selected from at least one of W, Mo, Ni, and Co, and the temperature of the contact reaction is within the range of 100-240°C.

The invention unexpectedly improves the selectivity of 2-methylpyridine while obtaining the high conversion rate of aniline by using a β zeolite carrying an active metal component selected from at least one of W, Mo, Ni, and Co in a hydrogen-containing atmosphere, and controlling the reaction temperature to be lower than the temperature generally required by the reaction for synthesizing diphenylamine from aniline. Moreover, the method can realize the selective production of diphenylamine and 2-methylpyridine by adjusting the reaction temperature and using the same set of devices with the same catalyst.

According to a preferred embodiment of the invention, the active metal component in terms of metal element is contained in an amount of 0.5-5wt.%, preferably 0.5-3wt.%, based on the total amount of the catalyst.

Preferably, the metal component further comprises an auxiliary metal component selected from at least one of Li, Na, K, Mg, and Ca, the auxiliary metal component in terms of an oxidation state is contained in an amount of 0-6.5wt.%, preferably 0.5-5.5wt.%, based on the total amount of the catalyst.

By adding the auxiliary metal component, both the acid distribution on the surface of the catalyst and the pore structure of the catalyst can be improved, and both the conversion rate of aniline and the selectivity of a target product are further increased.

Although the mechanism is not clear, the present inventors have discovered that the above-mentioned effect of producing diphenylamine or 2-methylpyridine as the main product by adjusting the temperature can only be obtained by loading the above-mentioned metal component on a β zeolite under a hydrogen-containing atmosphere, the above-mentioned effect cannot be achieved by using other molecular sieves such as ZSM-5.

Preferably, the β zeolite has a Si/Al molar ratio within the range of 25-300, preferably within the range of 60-220.

Preferably, the catalyst has a specific surface area of 400-700$m^2$/g, more preferably 450-650$m^2$/g; a pore volume of 0.25-0.6mL/g, more preferably 0.4-0.55 mL/g; an average pore diameter of 1.5-5nm, more preferably 2-4nm; and a particle size preferably less than 9 nm.

The catalyst may further comprise an alumina binder, thereby obtaining a shaped body of the catalyst.

According to a preferred embodiment of the invention, the catalyst comprises 50-85wt.% of zeolite, 0.5-5wt.% of an active metal component in terms of the metal element, 0-6.5wt.% of an auxiliary metal component in terms of oxidation state, and 10-45wt.% of an alumina binder, based on the total amount of the catalyst. Preferably, the catalyst comprises 60-85wt.% of zeolite, 0.5-3wt.% of an active metal component in terms of the metal element, 0.5-5.5wt.% of an auxiliary metal component in terms of oxidation state, and 15-34.5wt.% of an alumina binder, based on the total amount of the catalyst.

Preferably, the catalyst is consisting of strip-shaped or spherical particles; when the catalyst is consisting of strip-shaped particles, the cross-section of the catalyst can be cylindrical, cloverleaf-shaped or four-leaf clover-shaped, and the cross-section width of the strip-shaped particle is within the range of 0.5-3.0mm, preferably within the range of 1.0-2.0 mm; when the catalyst is consisting of spherical particles, the particles have a diameter within the range of 0.5-5.0mm, preferably within the range of 1.0-3.0mm.

The above-mentioned catalysts can be prepared with the methods generally known by those skilled in the art or are commercially available. For example, the catalyst of the present invention can be prepared with the following method: (1) contacting Hβ zeolite with an aqueous nitrate solution of an auxiliary metal component in an equivalent-volume impregnation mode; (2) filtering, washing, and drying the mixture obtained in step (1); (3) fully kneading the modified Hβ zeolite obtained in step (2), an alumina binder and processing auxiliary agents (e.g., an extrusion auxiliary agent and a peptizing agent), molding, and then drying and roasting; (4) adding $NaBH_4$ into the carrier obtained in step (3) and an aqueous nitrate solution of an active metal component for reflux reduction, filtering, and drying in vacuum to prepare the catalyst.

Wherein the contact temperature in step (1) is within the range of 80-100°C, and the contact time is 3-6 h; drying in step (2) is performed at 60-120°C for 6-12h; drying in step (3) is performed at 60-120°C for 6-12h; roasting is performed at the temperature of 450-550°C for 4-16h; the reflux reduction in step (4) is implemented at the temperature of 80-100°C for 6-8h, and the vacuum drying is then carried out at the temperature of 80-100°C for 6-12h.

By using the above catalyst, the invention can obtain a high yield of 2-methylpyridine at a lower temperature under a hydrogen-containing atmosphere rather than the traditional ammonia gas atmosphere. Therefore, the contact reaction is preferably performed under the circumstance without introducing ammonia gas. The hydrogen-containing atmosphere refers to the concentration of hydrogen gas within the range of 10-100 vol.%.

Further, the temperature of the contact reaction is within the range of 100-240°C. The specific reaction temperature may be 100°C, 110°C, 120°C, 130°C, 140°C, 150°C, 160°C, 170°C, 180°C, 190°C, 199°C, 200°C, 210°C, 220°C, 230°C, 240°C, and any value within the range consisting of any two of the point values. The preferable reaction temperature may be within the range of 130-180°C, the specific reaction temperature may be 130°C, 140°C, 150°C, 160°C, 170°C, 180°C, and any value within the range consisting of any two of the point values.

The method of the present invention may be carried out continuously or intermittently and is preferably carried out continuously. The intermittent operation is usually a kettle-type operation, and the target product can be obtained by adding the aniline, and the catalyst into a reaction kettle and carrying out the reaction under appropriate conditions. The continuous operation generally refers to the aniline is reacted through a fixed bed reactor containing the catalyst.

The present inventors have found that the optimum reaction temperature and pressure are slightly different between the continuous reaction and the intermittent reaction. When the intermittent reaction is adopted, the temperature of the contact reaction is preferably within the range of 130-180°C; the pressure of the contact reaction is preferably within the range of 2-4MPa, and the reaction time is preferably within the range of 4-8h; the used amount of the catalyst preferably accounts for 1-4 wt.% of the used amount of the aniline. When the continuous reaction is adopted, the temperature of the contact reaction is preferably within the range of 160-240°C; the pressure of the contact reaction is preferably within the range of 1.5-3MPa, and the liquid hourly mass space velocity of the aniline is preferably within the range of 0.5-3$h^{-1}$, further preferably 1-1.5$h^{-1}$.

The continuous operation is preferably carried out by feeding the aniline into the bottom of the fixed bed reactor and passing through a catalyst bed of the fixed bed reactor comprising the catalyst.

The present inventors have also found that the reaction for generating 2-methylpyridine by isomeric rearrangement of aniline and the reaction for preparing diphenylamine by condensation of aniline are influenced by the two factors of catalyst and reaction temperature. Use of the metal-loaded β zeolite catalyst of the present invention, the reaction route of aniline can be switched between 2-methylpyridine and diphenylamine due to the change of the catalytic activity center. Moreover, the low reaction temperature is more favorable for synthesizing 2-methylpyridine, and the high temperature is conducive to the generation of diphenylamine.

Therefore, the invention also provides a method for selectively and continuously producing 2-methylpyridine and diphenylamine from aniline, the method comprises: in a hydrogen-containing atmosphere, enabling an aniline and a catalyst to undergo a contact reaction at a temperature of 100-400°C, wherein the catalyst is zeolite carrying a metal component, and the metal component comprises an active metal component selected from at least one of W, Mo, Ni, and Co; controlling the temperature of the contact reaction to be 100-240°C to obtain a product mainly consisting of 2-methylpyridine; and controlling the temperature of the contact reaction to be 260-400°C to obtain a product mainly consisting of diphenylamine.

Preferably, when a product mainly containing 2-methylpyridine is obtained, the temperature of the contact reaction is within the range of 160-240°C, the pressure of the contact reaction is within the range of 1.5-3MPa, and the liquid hourly mass space velocity of the aniline is preferably within the range of 0.5-3$h^{-1}$; when a product mainly consisting of diphenylamine is obtained, the temperature of the contact reaction within the range of 280-360°C, the pressure of the contact reaction is within the range of 1.5-3MPa, and the liquid hourly mass space velocity of the aniline is preferably within the range of 0.3-1.5$h^{-1}$.

In the invention, the product mainly containing 2-methylpyridine refers to that the selectivity of 2-methylpyridine is not less than 40%, and the product mainly containing diphenylamine refers to the selectivity of diphenylamine is not less than 40%.

The catalyst is described above and will not be repeatedly described herein.

The conditions for synthesizing diphenylamine with aniline in the invention comprise: the temperature of the contact reaction is preferably within the range of 280-360°C, the pressure of the contact reaction is preferably within the range of 1.5-3MPa, and the liquid hourly mass space velocity of the aniline is preferably within the range of 0.3-1.5$h^{-1}$, further preferably 0.5-1$h^{-1}$.

In the Examples and Comparative Examples, the calculation methods of the conversion rate of aniline, the selectivity and yield of 2-methylpyridine, and the selectivity and yield of diphenylamine are as follows:

The conversion rate of aniline = (mole number of aniline in starting material before reaction - mole number of aniline in the product) / mole number of aniline in starting material before reaction ×100%;

The yield of 2-methylpyridine = mole number of 2-methylpyridine in the product/mole number of all the aniline in starting material before reaction theoretically converted into 2-methylpyridine;

The selectivity of 2-methylpyridine = (mole number of 2-methylpyridine in the product)/(mole number of aniline in starting material before reaction - mole number of aniline in the product) ×100%;

The yield of diphenylamine = molar number of diphenylamine in the product/molar number of all the aniline in the starting material before the reaction theoretically converted into diphenylamine;

The selectivity of diphenylamine =2× (mole number of diphenylamine in the product)/(mole number of aniline in the starting material before reaction - mole number of aniline in the product) ×100%.

The contents of aniline, diphenylamine, and 2-methylpyridine were measured according to gas chromatography.

The alumina binder in use was a commercially available γ-alumina, and the zeolite in use was a commercially available Hβ zeolite having a specific surface area of 620m$^2$/g and a particle size less than 9nm.

The catalysts used in the Examples and Comparative Examples were prepared with the method of loading auxiliary metal component and active metal component by the saturated impregnation method, and the concrete method was as follows: (1) Hβ zeolite contacted with an aqueous nitrate solution of an auxiliary metal component in an equivalent-volume impregnation mode; (2) the mixture obtained in step (1) was subjected to filtering, washing, and drying; (3) the modified Hβ zeolite obtained in step (2), an alumina binder (e.g., Pseudo-Boehmite) and processing auxiliary (e.g., an extrusion auxiliary and a peptizing agent) were subjected to fully kneading, molding, and then drying and roasting; (4) $NaBH_4$ was added into the carrier obtained in step (3) and an aqueous nitrate solution of an active metal component for reflux reduction, then subjected to filtering, and drying in vacuum to prepare the spherical catalyst with the particle diameter of 3 mm. Wherein the contact temperature in step (1) was 90°C, and the contact time was 4h; drying in step (2) was performed at 80°C for 10h; drying in step (3) was performed at 80°C for 10h; roasting was performed at the temperature of 550°C for 10h; the reflux reduction in step (4) was implemented at the temperature of 100°C for 8h, and the vacuum drying was then carried out at the temperature of 100°C for 10h.

The properties of the catalysts were listed in Table 1.

Table 1 Catalysts

| | | Catalysts | | | | | | | | |
|---|---|---|---|---|---|---|---|---|---|---|
| | | A | B | C | D | E | F | G | H | I |
| Active metal | Types | Ni | Ni | Ni | Mo | Ni | Co | Ni | - | Ni |
| | Content (wt.%) | 0.5 | 1.5 | 2 | 1.5 | 5 | 1.5 | 1.5 | - | 1.5 |
| Hβ zeolite (wt.%) | | 60 | 82 | 80 | 80 | 75 | 80 | 82 | 82 | ZSM-5 |
| Si/Al ratio of Hβ zeolite | | 60 | 60 | 200 | 60 | 60 | 60 | 60 | 60 | - |
| Auxiliary metal | Types | K | Mg | K | Na | Ca | Na | - | Mg | Mg |
| | Content in terms of | 5.2 | 1 | 0.5 | 3 | 2 | 3 | - | 1 | 1 |
| | oxidation state (wt.%) | | | | | | | | | |
| Alumina | | Balance | Balance | Balance | Balance | Balance | Balance | Balance | Balance | Balance |

Examples 1-8

A kettle-type reactor was selected, the reaction was carried out in a hydrogen atmosphere, the used amount of the catalyst was 1g, the added amount of the aniline was 50g, and the reaction conditions were as shown in Table 2 below.

Comparative Examples 1-2

The conversion reaction of aniline was carried out according to the method of Example 1, except that the reaction conditions were as shown in Table 2 below.

Comparative Example 3

The conversion reaction of aniline was carried out according to the method of Example 1, except that β zeolite (metal component was not loaded) was used as the catalyst, and other reaction conditions were shown in Table 2 below.

Comparative Example 4

The production of 2-methylpyridine from aniline was performed according to the method in Example 1 of CA1190928A as follows: pure HZSM-5 was used as the catalyst for converting the aniline into the 2-methylpyridine under the conditions consisting of a temperature of 510°C, a pressure of 2.9MPa, molar ratio of the aniline to ammonia of 1: 8, and the mass space velocity of $1.0h^{-1}$.

Table 2

| | | Catalysts | Reaction temperature (°C) | Reaction pressure (MPa) | Reaction time (h) | Conversion rate of aniline (mol%) | The yield of 2-methylpyridine (mol%) | The selectivity of 2-methylpyridine (mol%) |
|---|---|---|---|---|---|---|---|---|
| Examples | 1 | A | 130 | 4 | 6 | 40.2 | 24.4 | 60.7 |
| | 2 | B | 180 | 4 | 6 | 45.4 | 25.7 | 56.6 |
| | 3 | C | 200 | 3 | 6 | 46.2 | 20.9 | 45.3 |
| | 4 | D | 100 | 2 | 4 | 36.7 | 22.5 | 61.3 |
| | 5 | E | 130 | 4 | 6 | 37.6 | 21.6 | 57.4 |
| | 6 | F | 150 | 2 | 8 | 42.4 | 24.6 | 58.1 |
| | 7 | B | 220 | 4 | 6 | 47.4 | 23.4 | 49.3 |
| | 8 | G | 180 | 4 | 6 | 33.2 | 16.0 | 48.3 |
| Comparative Examples | 1 | I | 200 | 3 | 6 | 15.5 | 3.9 | 25.3 |
| | 2 | A | 260 | 4 | 6 | 46.3 | 14.2 | 30.7 |
| | 3 | H | 180 | 4 | 6 | 20.6 | 0.8 | 3.9 |
| | 4 | ZSM-5 | 510 | 2.9 | 1 | 13.7 | 7.1 | 51.6 |

Examples 9-16

A fixed bed reactor was selected, and the aniline passed through a catalyst bed in the lower feeding mode, the reaction was firstly carried out for 48 hours under the condition suitable for generating 2-methylpyridine in a hydrogen atmosphere, then the reaction was continued for 48 hours by switching to the condition suitable for generating diphenylamine at the temperature rise rate of 30°C/hour, and the measured product distribution was shown in the following Table 3.

Table 3

| | Examples | | | | | | | |
|---|---|---|---|---|---|---|---|---|
| | 9 | 10 | 11 | 12 | 13 | 14 | 15 | 16 |
| Before 48h | | | | | | | | |
| Catalysts | B | B | C | D | C | C | A | G |
| Reaction temperature (°C) | 160 | 220 | 200 | 200 | 240 | 260 | 160 | 160 |
| Reaction pressure (MPa) | 1.5 | 3 | 2 | 3 | 2 | 2 | 1.5 | 1.5 |
| Liquid hourly mass space velocity ($h^{-1}$) | 1.5 | 1.5 | 1 | 1.5 | 1 | 1 | 1.5 | 1.5 |
| The conversion rate of aniline (mol%) | 33.5 | 34.7 | 38.1 | 37.7 | 38.3 | 38.9 | 30.6 | 26.7 |
| The yield of 2-methylpyridine (mol%) | 22.6 | 21.8 | 24.6 | 22.1 | 21 | 18.4 | 20.3 | 16.9 |
| Selectivity of 2-methylpyridine (mol%) | 67.6 | 62.9 | 64.5 | 58.6 | 54.8 | 47.4 | 66.3 | 63.4 |
| 48-96 hours | | | | | | | | |
| catalyst | B | B | C | D | C | C | A | G |
| Reaction temperature (°C) | 280 | 315 | 360 | 340 | 295 | 340 | 280 | 280 |
| Reaction pressure (MPa) | 2 | 1.5 | 3 | 2 | 1.5 | 2 | 2 | 2 |
| Liquid hourly mass space velocity ($h^{-1}$) | 0.5 | 1 | 0.8 | 1 | 0.9 | 1 | 0.5 | 0.5 |
| The conversion rate of aniline (mol%) | 39.2 | 39.8 | 42.1 | 41.3 | 38.1 | 40.7 | 37.8 | 33.2 |
| Selectivity of diphenylamine (mol%) | 49.4 | 50.3 | 56.6 | 51.2 | 53.3 | 55.7 | 53.5 | 43.6 |

Example 17

Diphenylamine and 2-methylpyridine were produced from aniline according to the method of Example 9, in order to examine the influence of temperature on the distribution of products, and after each sampling, the temperature was raised at a temperature-rise rate of 30°C/hour until the next temperature, and the graph of the product distribution change along with the temperature was shown in FIG. 1.

As can be seen from the results of Table 2 and FIG. 1, the method for preparing 2-methylpyridine provided by the invention can obtain the high conversion rate of aniline and high selectivity of 2-methylpyridine at a low reaction temperature.

It can be derived from the results of Table 3 and FIG. 1 that the method for selectively and continuously producing diphenylamine and 2-methylpyridine provided by the invention can conveniently produce different target products diphenylamine and 2-methylpyridine by adjusting the reaction temperature, pressure, and other process parameters on a set of apparatus.

## Claims

1. A method for selectively and continuously producing 2-methylpyridine and diphenylamine from aniline, the method comprises: in a hydrogen-containing atmosphere, enabling an aniline and a catalyst to undergo a contact reaction at a temperature of 100-400°C, wherein the catalyst is a β zeolite carrying a metal component, and the metal component comprises an active metal component selected from at least one of W, Mo, Ni, and Co; controlling the temperature of the contact reaction to be 100-240°C to obtain a product mainly consisting of 2-methylpyridine; and controlling the temperature of the contact reaction to be 260-400°C to obtain a product mainly consisting of diphenylamine.

2. The method according to claim 1, wherein the active metal component in terms of metal element is contained in an amount of 0.5-5wt.%, preferably 0.5-3wt.%, based on the total amount of the catalyst.

3. The method according to claim 1 or 2, wherein the metal component further comprises an auxiliary metal component selected from at least one of Li, Na, K, Mg, and Ca, the auxiliary metal component in terms of an oxidation state is contained in an amount of 0-6.5wt.%, preferably 0.5-5.5wt.%, based on the total amount of the catalyst.

4. The method according to any one of claims 1-3, wherein the catalyst further comprises an alumina binder, the catalyst comprises 50-85wt.% of a β zeolite, 0.5-5wt.% of an active metal component in terms of metal element, 0-6.5wt.% of an auxiliary metal component in terms of oxidation state, and 10-45wt.% of an alumina binder, based on the total amount of the catalyst.

5. The method according to claim 4, wherein the catalyst comprises 60-85wt.% of a β zeolite, 0.5-3wt.% of an active metal component in terms of the metal element, 0.5-5.5wt.% of an auxiliary metal component in terms of oxidation state, and 15-34.5wt.% of an alumina binder, based on the total amount of the catalyst.

6. The method according to any one of claims 1-5, wherein the pressure of the contact reaction is within a range of 1.5-4MPa, the contact reaction is carried out under a hydrogen atmosphere.

7. The method according to any one of claims 1-6, wherein the contact reaction is performed under the circumstance without introducing ammonia gas.

8. The method according to any one of claims 1-7, when a product mainly consisting of 2-methylpyridine is obtained, the temperature of the contact reaction is within the range of 160-240°C, the pressure of the contact reaction is within the range of 1.5-3MPa, and the liquid hourly mass space velocity of the aniline is preferably within the range of 0.5-3$h^{-1}$; or when a product mainly consisting of diphenylamine is obtained, the temperature of the contact reaction is within the range of 280-360°C, the pressure of the contact reaction is within the range of 1.5-3MPa, and the liquid hourly mass space velocity of the aniline is preferably within the range of 0.3-1.5$h^{-1}$.

9. The method according to claim 8, wherein the aniline is reacted through a fixed bed reactor containing the catalyst; preferably, the aniline is fed from the bottom of the fixed bed reactor and passes through a catalyst bed.

10. A method for synthesizing 2-methylpyridine from aniline, wherein the method comprises carrying out a contact reaction between an aniline and a catalyst under a hydrogen-containing atmosphere and a condition for isomeric rearrangement reaction, the catalyst is a β zeolite carrying a metal component comprising an active metal component selected from at least one of W, Mo, Ni, and Co, and the temperature of the contact reaction is within the range of 100-240°C.

11. The method according to claim 10, wherein the active metal component in terms of metal element is contained in an amount of 0.5-5wt.%, preferably 0.5-3wt.%, based on the total amount of the catalyst.

12. The method according to claim 10 or 11, wherein the metal component further comprises an auxiliary metal component selected from at least one of Li, Na, K, Mg, and Ca, the auxiliary metal component in terms of an oxidation state is contained in an amount of 0-6.5wt.%, based on the total amount of the catalyst.

13. The method according to any one of claims 10-12, wherein the catalyst further comprises an alumina binder, the catalyst comprises 50-85wt.% of a β zeolite, 0.5-5wt.% of an active metal component in terms of metal element, 0-6.5wt.% of an auxiliary metal component in terms of oxidation state, and 10-45wt.% of an alumina binder, based on the total amount of the catalyst.

14. The method according to claim 13, wherein the catalyst comprises 60-85wt.% of a β zeolite, 0.5-3wt.% of an active metal component in terms of reduced state, 0.5-5.5wt.% of an auxiliary metal component in terms of oxidation state, and 15-34.5wt.% of an alumina binder, based on the total amount of the catalyst.

15. The method according to any one of claims 10-14, wherein the contact reaction is performed under the circumstance without introducing ammonia gas;

preferably, the contact reaction is carried out intermittently, the temperature of the contact reaction is within the

range of 130-180°C, the pressure of the contact reaction is within the range of 2-4MPa, the reaction time is within the range of 4-8h, and preferably, the used amount of the catalyst accounts for 1-4wt.% of the used amount of the aniline;

preferably,the contact reaction is carried out continuously, the temperature of the contact reaction is within the range of 160-240°C, the pressure of the contact reaction is within the range of 1.5-3MPa, and the liquid hourly mass space velocity of the aniline is preferably within the range of 0.5-3$h^{-1}$;

preferably, the aniline is reacted through a fixed bed reactor containing the catalyst, and more preferably, the aniline is fed from the bottom of the fixed bed reactor and passes through a catalyst bed.

**Patentansprüche**

1. Verfahren zur selektiven und kontinuierlichen Herstellung von 2-Methylpyridin und Diphenylamin aus Anilin, wobei das Verfahren umfasst: in einer wasserstoffhaltigen Atmosphäre Anilin und einem Katalysator eine Kontaktreaktion zu ermöglichen bei einer Temperatur von 100-400 °C, wobei der Katalysator ein β-Zeolith ist, der eine Metallkomponente trägt, und die Metallkomponente eine aktive Metallkomponente umfasst, mindestens eine ausgewählt aus W, Mo, Ni und Co; Einstellen der Temperatur der Kontaktreaktion auf 100-240 °C, um ein Produkt zu erhalten, das hauptsächlich aus 2-Methylpyridin besteht; und Einstellen der Temperatur der Kontaktreaktion auf 260-400 °C, um ein Produkt zu erhalten, das hauptsächlich aus Diphenylamin besteht.

2. Verfahren nach Anspruch 1, wobei die aktive Metallkomponente, bezogen auf das Metallelement, in einer Menge von 0,5-5 Gew.-%, vorzugsweise 0,5-3 Gew.-%, bezogen auf die Gesamtmenge des Katalysators, enthalten ist.

3. Verfahren nach Anspruch 1 oder 2, wobei die Metallkomponente ferner eine Hilfsmetallkomponente umfasst, die mindestens eine ist, ausgewählt aus Li, Na, K, Mg und Ca, wobei die Hilfsmetallkomponente, bezogen auf den oxidierten Zustand, in einer Menge von 0-6,5 Gew.-%, vorzugsweise 0,5-5,5 Gew.-%, bezogen auf die Gesamtmenge des Katalysators, enthalten ist.

4. Verfahren nach einem der Ansprüche 1-3, wobei der Katalysator ferner ein Aluminiumoxid-Bindemittel umfasst, der Katalysator umfasst 50-85 Gew.-% eines β-Zeoliths, 0,5-5 Gew.-% einer aktiven Metallkomponente, bezogen auf das Metallelement, 0-6,5 Gew.-% einer Hilfsmetallkomponente, bezogen auf den oxidierten Zustand, und 10-45 Gew.-% eines Aluminiumoxid-Bindemittels, bezogen auf die Gesamtmenge des Katalysators.

5. Verfahren nach Anspruch 4, wobei der Katalysator 60-85 Gew.-% eines β-Zeoliths, 0,5-3 Gew.-% einer aktiven Metallkomponente, bezogen auf das Metallelement, 0,5-5,5 Gew.-% einer Hilfsmetallkomponente, bezogen auf den oxidierten Zustand, und 15-34,5 Gew.-% eines Aluminiumoxid-Bindemittels, bezogen auf die Gesamtmenge des Katalysators, umfasst.

6. Verfahren nach einem der Ansprüche 1-5, wobei der Druck der Kontaktreaktion im Bereich von 1,5-4 MPa liegt und die Kontaktreaktion unter einer Wasserstoffatmosphäre durchgeführt wird.

7. Verfahren nach einem der Ansprüche 1-6, wobei die Kontaktreaktion unter Bedingungen durchgeführt wird, bei denen kein Ammoniakgas zugeführt wird.

8. Verfahren nach einem der Ansprüche 1-7, wobei, wenn ein hauptsächlich aus 2-Methylpyridin bestehendes Produkt erhalten wird, die Temperatur der Kontaktreaktion im Bereich von 160-240 °C liegt, der Druck der Kontaktreaktion im Bereich von 1,5-3 MPa liegt und die stündliche Massenraumgeschwindigkeit des Anilins vorzugsweise im Bereich von 0,5-3 $h^{-1}$ liegt; oder

   wenn ein Produkt erhalten wird, das hauptsächlich aus Diphenylamin besteht, die Temperatur der Kontaktreaktion im Bereich von 280-360 °C liegt, der Druck der Kontaktreaktion im Bereich von 1,5-3 MPa liegt und die stündliche Massenraumgeschwindigkeit des Anilins vorzugsweise im Bereich von 0,3-1,5 $h^{-1}$ liegt.

9. Verfahren nach Anspruch 8, wobei das Anilin in einem den Katalysator enthaltenden Festbettreaktor zur Reaktion gebracht wird;

   vorzugsweise wird das Anilin vom Boden des Festbettreaktors zugeführt und durchläuft ein Katalysatorbett.

10. Verfahren zur Synthese von 2-Methylpyridin aus Anilin, wobei das Verfahren die Durchführung einer Kontaktreaktion zwischen Anilin und einem Katalysator unter einer wasserstoffhaltigen Atmosphäre und unter Bedingungen für eine

isomere Umlagerungsreaktion umfasst, wobei der Katalysator ein β-Zeolith ist, der eine Metallkomponente trägt, die eine aktive Metallkomponente umfasst, die mindestens eine ist ausgewählt aus W, Mo, Ni und Co, und die Temperatur der Kontaktreaktion im Bereich von 100-240 °C liegt.

**11.** Verfahren nach Anspruch 10, wobei die aktive Metallkomponente, bezogen auf das Metallelement, in einer Menge von 0,5-5 Gew.-%, vorzugsweise 0,5-3 Gew.-%, bezogen auf die Gesamtmenge des Katalysators, enthalten ist.

**12.** Verfahren nach Anspruch 10 oder 11, wobei die Metallkomponente ferner eine Hilfsmetallkomponente umfasst, die mindestens eine ist, ausgewählt aus Li, Na, K, Mg und Ca, und die Hilfsmetallkomponente, bezogen auf den oxidierten Zustand, in einer Menge von 0-6,5 Gew.-%, bezogen auf die Gesamtmenge des Katalysators, enthalten ist.

**13.** Verfahren nach einem der Ansprüche 10-12, wobei der Katalysator ferner ein Aluminiumoxid-Bindemittel umfasst, der Katalysator umfasst 50-85 Gew.-% eines β-Zeoliths, 0,5-5 Gew.-% einer aktiven Metallkomponente, bezogen auf das Metallelement, 0-6,5 Gew.-% einer Hilfsmetallkomponente, bezogen auf den oxidierten Zustand, und 10-45 Gew.-% eines Aluminiumoxid-Bindemittels, bezogen auf die Gesamtmenge des Katalysators.

**14.** Verfahren nach Anspruch 13, wobei der Katalysator 60-85 Gew.-% eines β-Zeoliths, 0,5-3 Gew.-% einer aktiven Metallkomponente, bezogen auf den reduzierten Zustand, 0,5-5,5 Gew.-% einer Hilfsmetallkomponente, bezogen auf den oxidierten Zustand, und 15-34,5 Gew.-% eines Aluminiumoxid-Bindemittels, bezogen auf die Gesamtmenge des Katalysators, umfasst.

**15.** Verfahren nach einem der Ansprüche 10-14, wobei die Kontaktreaktion unter Bedingungen durchgeführt wird, bei denen kein Ammoniakgas zugeführt wird;

vorzugsweise wird die Kontaktreaktion diskontinuierlich durchgeführt, die Temperatur der Kontaktreaktion liegt im Bereich von 130-180 °C, der Druck der Kontaktreaktion liegt im Bereich von 2-4 MPa, die Reaktionszeit liegt im Bereich von 4-8 h, und vorzugsweise beträgt die eingesetzte Menge des Katalysators 1-4 Gew.-% der eingesetzten Menge des Anilins;
vorzugsweise wird die Kontaktreaktion kontinuierlich durchgeführt, die Temperatur der Kontaktreaktion liegt im Bereich von 160-240 °C, der Druck der Kontaktreaktion liegt im Bereich von 1,5-3 MPa, und die stündliche Massenraumgeschwindigkeit des Anilins liegt vorzugsweise im Bereich von 0,5-3 $h^{-1}$;
vorzugsweise wird das Anilin in einem den Katalysator enthaltenden Festbettreaktor zur Reaktion gebracht, und mehr bevorzugt wird das Anilin vom Boden des Festbettreaktors zugeführt und durchläuft ein Katalysatorbett.

## Revendications

**1.** Procédé de production sélective et continue de 2-méthylpyridine et de diphénylamine à partir d'aniline, le procédé comprenant : dans une atmosphère contenant de l'hydrogène, le fait de permettre à une aniline et à un catalyseur de subir une réaction de contact à une température de 100 à 400 °C, le catalyseur étant une zéolite β portant un composant métallique, et le composant métallique comprenant un composant métallique actif choisi parmi au moins l'un parmi W, Mo, Ni et Co ; la régulation de la température de la réaction de contact pour qu'elle soit de 100 à 240 °C pour obtenir un produit constitué principalement de 2-méthylpyridine ; et la régulation de la température de la réaction de contact pour qu'elle soit de 260 à 400 °C pour obtenir un produit constitué principalement de diphénylamine.

**2.** Procédé selon la revendication 1, dans lequel le composant métallique actif en termes d'élément métallique est contenu en une quantité de 0,5 à 5 % en poids, de préférence de 0,5 à 3 % en poids, sur la base de la quantité totale du catalyseur.

**3.** Procédé selon la revendication 1 ou 2, dans lequel le composant métallique comprend en outre un composant métallique auxiliaire choisi parmi au moins l'un parmi Li, Na, K, Mg et Ca, le composant métallique auxiliaire en termes d'état d'oxydation étant contenu en une quantité de 0 à 6,5 % en poids, de préférence de 0,5 à 5,5 % en poids, sur la base de la quantité totale du catalyseur.

**4.** Procédé selon l'une quelconque des revendications 1 à 3, dans lequel le catalyseur comprend en outre un liant d'alumine, le catalyseur comprend de 50 à 85 % en poids d'une zéolite β, de 0,5 à 5 % en poids d'un composant métallique actif en termes d'élément métallique, de 0 à 6,5 % en poids d'un composant métallique auxiliaire en termes d'état d'oxydation, et de 10 à 45 % en poids d'un liant d'alumine, sur la base de la quantité totale du catalyseur.

5. Procédé selon la revendication 4, dans lequel le catalyseur comprend de 60 à 85 % en poids d'une zéolite β, de 0,5 à 3 % en poids d'un composant métallique actif en termes d'élément métallique, de 0,5 à 5,5 % en poids d'un composant métallique auxiliaire en termes d'état d'oxydation, et de 15 à 34,5 % en poids d'un liant d'alumine, sur la base de la quantité totale du catalyseur.

6. Procédé selon l'une quelconque des revendications 1 à 5, dans lequel la pression de la réaction de contact se situe dans une plage de 1,5 à 4 MPa, la réaction de contact étant effectuée sous une atmosphère d'hydrogène.

7. Procédé selon l'une quelconque des revendications 1 à 6, dans lequel la réaction de contact est effectuée sans introduction de gaz ammoniac.

8. Procédé selon l'une quelconque des revendications 1 à 7, lorsqu'un produit constitué principalement de 2-méthylpyridine est obtenu, la température de la réaction de contact se situe dans la plage de 160 à 240 °C, la pression de la réaction de contact se situe dans la plage de 1,5 à 3 MPa, et la vitesse spatiale massique horaire liquide de l'aniline se situe de préférence dans la plage de 0,5 à 3 $h^{-1}$ ; ou lorsqu'un produit constitué principalement de diphénylamine est obtenu, la température de la réaction de contact se situe dans la plage de 280 à 360 °C, la pression de la réaction de contact se situe dans la plage de 1,5 à 3 MPa, et la vitesse spatiale massique horaire liquide de l'aniline se situe de préférence dans la plage de 0,3 à 1,5 $h^{-1}$.

9. Procédé selon la revendication 8, dans lequel l'aniline est mise en réaction à travers un réacteur à lit fixe contenant le catalyseur ;
de préférence, l'aniline est introduite par le bas du réacteur à lit fixe et traverse un lit de catalyseur.

10. Procédé de synthèse de 2-méthylpyridine à partir d'aniline, dans lequel le procédé comprend la réalisation d'une réaction de contact entre une aniline et un catalyseur sous une atmosphère contenant de l'hydrogène et une condition de réaction de réarrangement isomérique, le catalyseur est une zéolite β portant un composant métallique comprenant un composant métallique actif choisi parmi au moins l'un parmi W, Mo, Ni et Co, et la température de la réaction de contact se situe dans la plage de 100 à 240 °C.

11. Procédé selon la revendication 10, dans lequel le composant métallique actif en termes d'élément métallique est contenu en une quantité de 0,5 à 5 % en poids, de préférence de 0,5 à 3 % en poids, sur la base de la quantité totale du catalyseur.

12. Procédé selon la revendication 10 ou 11, dans lequel le composant métallique comprend en outre un composant métallique auxiliaire choisi parmi au moins l'un parmi Li, Na, K, Mg et Ca, le composant métallique auxiliaire en termes d'état d'oxydation étant contenu en une quantité de 0 à 6,5 % en poids, sur la base de la quantité totale du catalyseur.

13. Procédé selon l'une quelconque des revendications 10 à 12, dans lequel le catalyseur comprend en outre un liant d'alumine, le catalyseur comprend de 50 à 85 % d'une zéolite β, de 0,5 à 5 % en poids d'un composant métallique actif en termes d'élément métallique, de 0 à 6,5 % d'un composant métallique auxiliaire en termes d'état d'oxydation, et de 10 à 45 % en poids d'un liant d'alumine, sur la base de la quantité totale du catalyseur.

14. Procédé selon la revendication 13, dans lequel le catalyseur comprend de 60 à 85 % en poids d'une zéolite β, de 0,5 à 3 % en poids d'un composant métallique actif en termes d'état réduit, de 0,5 à 5,5 % en poids d'un composant métallique auxiliaire en termes d'état d'oxydation, et de 15 à 34,5 % en poids d'un liant d'alumine, sur la base de la quantité totale du catalyseur.

15. Procédé selon l'une quelconque des revendications 10 à 14, dans lequel la réaction de contact est effectuée sans introduction de gaz ammoniac ;

de préférence, la réaction de contact est effectuée de manière intermittente, la température de la réaction de contact se situe dans la plage de 130 à 180 °C, la pression de la réaction de contact se situe dans la plage de 2 à 4 MPa, le temps de réaction se situe dans la plage de 4 à 8 heures, et de préférence, la quantité utilisée du catalyseur représente de 1 à 4 % en poids de la quantité utilisée de l'aniline ;
de préférence, la réaction de contact est effectuée en continu, la température de la réaction de contact se situe dans la plage de 160 à 240 °C, la pression de la réaction de contact se situe dans la plage de 1,5 à 3 MPa, et la vitesse spatiale massique horaire liquide de l'aniline se situe de préférence dans la plage de 0,5 à 3 $h^{-1}$ ;
de préférence, l'aniline est mise en réaction à travers un réacteur à lit fixe contenant le catalyseur, et plus

préférablement, l’aniline est introduite par le bas du réacteur à lit fixe et passe à travers un lit de catalyseur.

Relationship between product distribution and temperature
Product distribution, mol%
80
70
60
50
40
30
20
10
0
100
150
200
250
300
350
400
Temperature/°C
Aniline conversion rate
2-methylpyridine selectivity
Diphenylamine selectivity

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111278790
- CN 105384683 A
- CA 1190928 A
- US 4395554 A
- US 4388461 A


### Non-patent literature cited in the description


- The zeolite-catalysed isomerization of aniline to α-picoline [J].. *Applied catalysis A: General*, 1998, vol. 172, 285-294